# EUROPEAN PATENT SPECIFICATION

(11) **EP 3 681 907 B1**
(45) Date of publication and mention of the grant of the patent: **02.12.2026**
(21) Application number: 18855393.7
(22) Date of filing: 13.09.2018
(51) Int. Cl.: G01N 33/68, C07K 16/06

(54) **IDENTIFICATION AND MONITORING OF IMMUNOGLOBULIN J CHAINS**
IDENTIFIZIERUNG UND ÜBERWACHUNG VON IMMUNGLOBULIN-J-KETTEN
IDENTIFICATION ET SURVEILLANCE DE CHAÎNES D'IMMUNOGLOBULINES J

(30) Priority: 13.09.2017 US 201762558023 P
(43) Date of publication of application: 22.07.2020
(73) Proprietor: Mayo Foundation for Medical Education and Research, Rochester, MN 55905 (US)
(72) Inventor: MURRAY, David L., Rochester, Minnesota 55906-4083 (US); BARNIDGE, David R., Rochester, Minnesota 55906 (US)
(74) Representative: Fish & Richardson P.C.
(86) International application number: PCT/US2018/050847
(87) International publication number: WO 2019/055632

(56) References cited:
- WO-A1-2015/154052
- US-A1- 2014 186 332
- US-A1- 2016 206 660
- US-A1- 2017 023 584
- L. S. MATHESON ET AL: "Light chain-deficient mice produce novel multimeric heavy-chain-only IgA by faulty class switching", INTERNATIONAL IMMUNOLOGY, vol. 21, no. 8, 1 August 2009 (2009-08-01), GB, pages 957 - 966, XP055305861, ISSN: 0953-8178, DOI: 10.1093/intimm/dxp062
- LIU YOUPING ET AL: "Analysis of plasma proteome from cases of the different traditional Chinese medicine syndromes in patients with chronic hepatitis B", JOURNAL OF PHARMACEUTICAL AND BIOMEDICAL ANALYSIS, ELSEVIER B.V, AMSTERDAM, NL, vol. 59, 8 October 2011 (2011-10-08), pages 173 - 178, XP028599149, ISSN: 0731-7085, DOI: 10.1016/J.JPBA.2011.10.002
- JOHANSEN ET AL: "Role of J Chain in Secretory Immunoglobulin Formation", SCANDINAVIAN JOURNAL OF IMMUNOLOGY, vol. 52, no. 3, 1 September 2000 (2000-09-01), GB, pages 240 - 248, XP055807363, ISSN: 0300-9475, DOI: 10.1046/j.1365-3083.2000.00790.x
- BAENZIGER JACQUES U: "Structure of the oligosaccharide of human J chain", JOURNAL OF BIOLOGICAL CHEMISTRY.(MICROFILMS), AMERICAN SOCIETY OF BIOLOGICAL CHEMISTS, BALTIMORE, MD, US, vol. 254, no. 10, 25 May 1979 (1979-05-25), pages 4063 - 4071, XP002973387
- HONG QIUTING ET AL: "A Method for Comprehensive Glycosite-Mapping and Direct Quantitation of Serum Glycoproteins", JOURNAL OF PROTEOME RESEARCH, vol. 14, no. 12, 9 November 2015 (2015-11-09), pages 5179 - 5192, XP055835324, ISSN: 1535-3893, DOI: 10.1021/acs.jproteome.5b00756

## Description

### CROss-REFERENCE To RELATED APPLICATIONS

This application claims the benefit of U.S. Patent Application Serial No. 62/558,023, filed on September 13, 2017.

### BACKGROUND

### 1. Technical Field

This document relates to materials and methods for identifying and quantifying immunoglobulin J chains in a sample, such as a biological sample, using a top-down mass spectrometry technique. In some cases, the methods are for diagnosing or monitoring a treatment of a disorder in a patient, wherein said disorder is associated with abnormal production of IgA immunoglobulins and/or IgM immunoglobulins.

### 2. Background Information

Human immunoglobulins contain two identical heavy chain polypeptides and two identical light chain polypeptides bound together by disulfide bonds. There are 2 different light chain isotypes (kappa and lambda) while there are 5 different heavy chain isotypes (IgG, IgA, IgM, IgD, and IgE). Each isotype has a unique amino acid sequence and set of post-translational modifications that are used to identify them.

A unique feature of immunoglobulins IgA and IgM is their ability to form multimers. In circulation IgA typically exists as a combination of monomers and dimers while IgM exists as a pentamer. IgA and IgM multimers form by connection through the J chain protein.

Matheson LS et al. describe in International Immunology, vol. 21, no. 8, 1 August 2009, on pages 957-966 that light chain-deficient mice produce novel multimeric heavy chain-only IgA by faulty class switching.

Quiting H et al. describe in Journal of Proteome Research, vol. 14, no. 12, 9 November 2015, on pages 5179-5192 a method for mapping of glycosylation sites and quantitation of serum glycoproteins.

### SUMMARY

The present invention is defined by the independent claims. The dependent claims depict additional embodiments of the invention.

This document provides materials and methods for identifying and quantifying immunoglobulin J chains in a sample, such as a biological sample, using top-down mass spectrometry (MS) techniques. For example, the materials and methods provided herein can be used to identify and quantify J chains. In some cases, identification and quantification of the J chain can be used to identify and quantify multimeric IgA immunoglobulins (e.g., IgA dimers) and/or multimeric IgM immunoglobulins (e.g., IgM pentamers). For example, the materials and methods provided herein can be used to identify and quantify posttranslational modifications (e.g., glycosylation) on the J chain.

As demonstrated herein, top-down MS can be used to analyze polyclonal and monoclonal IgA and IgM serum samples to obtain a comprehensive analysis of the J chain. For example, top-down MS can be used to identify and quantify the J chain associated multimeric IgA immunoglobulins (e.g., IgA dimers) and/or multimeric IgM immunoglobulins (e.g., IgM pentamers). Further, top-down MS also can be used to identify posttranslational modifications on the J chain such as glycosylation. Notably, the methods described herein can be done without the need for additional instrumentation and/or J chain specific reagents. Having the ability to identify and quantify the J chain associated with multimeric IgA immunoglobulins (e.g., IgA dimers) and/or multimeric IgM immunoglobulins (e.g., IgM pentamers), in a single assay, provides a unique and unrealized diagnostic tool to aid immunologists in monitoring a patient's immune system. For example, the J chain is associated with secretion, and accordingly, identifying and quantifying the J chain associated with multimeric IgA immunoglobulins and/or multimeric IgM immunoglobulins (e.g., IgA dimers and/or IgM pentamers from a patient serum sample) can be used to determine the amount of secretory IgA and/or IgM in a patient.

In general, one aspect of this document features a method for identifying J chains in a sample. The method includes, or consists essentially of, providing a sample comprising immunoglobulins, immunopurifying IgA immunoglobulins and/or IgM immunoglobulins from the sample, subjecting the immunopurified immunoglobulins to a top-down MS technique to obtain a mass spectrum of the sample, and identifying the presence of J chains based on the multiply charged ion peaks in the spectrum corresponding to the J chains. The immunoglobulins can be intact (e.g., not fragmented) during the top-down MS technique. The sample can be a biological sample (e.g., a biological fluid). The biological fluid can be blood, serum, plasma, urine, lachrymal fluid, or saliva. The biological fluid can be serum. The top-down MS technique can include a liquid chromatography-mass spectrometry (LC-MS) technique. The top-down MS technique can be electrospray ionization mass spectrometry (ESI-MS). The ESI-MS technique can include a quadrupole time-of- flight (TOF) mass spectrometer. The J chains can include a post-translational modification (e.g., glycosylation). The J chains can lack a post-translational modification. The immunopurifying can include using a non-human antibody (e.g., a camelid antibody, a cartilaginous fish antibody, llama, sheep, goat, rabbit, or a mouse antibody). The non-human antibody can be a camelid antibody. The antibody can be a single domain antibody fragment. The single domain antibody fragment can be derived from a camelid antibody.

In another aspect, this document features a method for quantifying the J chains in a sample. The method includes, or consists essentially of, providing a sample comprising immunoglobulins, immunopurifying IgA immunoglobulins comprising using an anti-human IgA antibody and/or IgM immunoglobulins comprising using an anti-human IgM antibody from the sample, subjecting the immunopurified immunoglobulins to a top-down MS technique to obtain a mass spectrum of the sample, identifying the presence of J chains based on the multiply charged ion peaks in the spectrum corresponding to the J chains, and converting the peak area of the identified peaks to a molecular mass to quantify the J chains in the sample. The method also can include immunopurifying IgA from the sample, where the immunopurifying includes using an anti-human IgA antibody. The method also can include immunopurifying IgM from the sample, where the immunopurifying can include using an anti-human IgM antibody. The immunopurifying can include using a non-human antibody (e.g., a camelid antibody, a cartilaginous fish antibody, llama, sheep, goat, rabbit, or a mouse antibody). The non-human antibody can be a camelid antibody. The antibody can be a single domain antibody fragment. The single domain antibody fragment can be derived from a camelid antibody. The method also can include immunopurifying IgA immunoglobulins from the sample, and further include quantifying the IgA immunoglobulins. The IgA immunoglobulins can be multimeric IgA immunoglobulins. The quantifying can include determining the ratio of J chains to IgA immunoglobulins. The method also can include immunopurifying IgM immunoglobulins from the sample, and further include quantifying the IgM immunoglobulins. The IgM immunoglobulins can be multimeric IgM immunoglobulins. The quantifying can include determining the ratio of J chains to IgM immunoglobulins. The immunoglobulins can be intact (e.g., not fragmented) during the top-down MS technique. The sample can be a biological sample (e.g., a biological fluid). The biological fluid can be blood, serum, plasma, urine, lachrymal fluid, or saliva. The biological fluid can be serum. The MS technique can include a liquid chromatography-mass spectrometry (LC- MS) technique. The top-down MS technique can be electrospray ionization mass spectrometry (ESI-MS). The ESI-MS technique can include a quadrupole time-of-flight (TOF) mass spectrometer. The J chains can include a post-translational modification (e.g., glycosylation). The J chains can lack a post-translational modification.

In another aspect, this document features a method for diagnosing a disorder in a patient, where the disorder is associated with abnormal production of IgA immunoglobulins and/or IgM immunoglobulins. The method includes, or consists essentially of, providing a sample comprising immunoglobulins from said patient, immunopurifying said IgA immunoglobulins and/or said IgM immunoglobulins from the sample, subjecting the immunopurified immunoglobulins to a top-down MS technique to obtain a mass spectrum of the sample, identifying the presence of J chains based on the multiply charged ion peaks in the spectrum corresponding to the J chains, determining the ratio of J chains to IgA immunoglobulins and/or the ratio of J chains to IgM immunoglobulins in the sample, and comparing the ratio to a reference value. The disorder can be multiple myeloma, asymptomatic or smoldering multiple myeloma, solitary plasmacytoma, monoclonal gammopathy of undetermined significance, B-cell chronic lymphocytic leukemia, Waldenstrom macroglobulinemia, non-secretory myeloma, selective IgA deficiency, selective IgM deficiency, celiac disease, IgA nephropathy, autoimmune disorders, or a neurologic disease. The patient can be a mammal (e.g., a human). The immunopurifying can include using a non-human antibody (e.g., a camelid antibody, a cartilaginous fish antibody, llama, sheep, goat, rabbit, or a mouse antibody). The non-human antibody can be a camelid antibody. The non-human antibody can be a single domain antibody fragment. The single domain antibody fragment can be derived from a camelid antibody. The immunoglobulins can be intact (e.g., not fragmented) during the top-down MS technique. The sample can be a biological sample (e.g., a biological fluid). The biological fluid can be blood, serum, plasma, urine, lachrymal fluid, or saliva. The biological fluid can be serum. The top-down MS technique can include a liquid chromatography-mass spectrometry (LC-MS) technique. The top-down MS technique can be electrospray ionization mass spectrometry (ESI-MS). The ESI-MS technique can include a quadrupole time-of- flight (TOF) mass spectrometer.

In another aspect, this document features a method for monitoring a treatment of a disorder in a patient, where the disorder is associated with abnormal production of IgA immunoglobulins and/or IgM immunoglobulins. The method includes, or consists essentially of, providing an initial sample comprising immunoglobulins from the patient, where the initial sample is obtained from the patient prior to the treatment, providing one or more secondary samples comprising immunoglobulins, where the one or more secondary samples are obtained from the patient during the treatment, after the treatment, or both, immunopurifying IgA immunoglobulins using an anti-human IgA antibody and/or IgM immunoglobulins using an anti-human IgM antibody from the samples, subjecting the immunopurified immunoglobulins to a top-down MS technique to obtain a mass spectrum of the samples, identifying the presence of J chains in said samples based on the multiply charged ion peaks in the spectrum corresponding to the J chains, determining the ratio of J chains to IgA immunoglobulins and/or the ratio of J chains to IgM immunoglobulins in the samples, and comparing the ratios from the initial sample and the one or more secondary samples. The disorder can be multiple myeloma, asymptomatic or smoldering multiple myeloma, solitary plasmacytoma, monoclonal gammopathy of undetermined significance, B-cell chronic lymphocytic leukemia, Waldenstrom macroglobulinemia, non-secretory myeloma, selective IgA deficiency, selective IgM deficiency, celiac disease, IgA nephropathy, autoimmune disorders, or a neurologic disease. The patient can be a mammal (e.g., a human). The immunopurifying can include using a non-human antibody (e.g., a camelid antibody, a cartilaginous fish antibody, llama, sheep, goat, rabbit, or a mouse antibody). The non-human antibody can be a camelid antibody. The non-human antibody can be a single domain antibody fragment. The single domain antibody fragment can be derived from a camelid antibody. The immunoglobulins can be intact (e.g., not fragmented) during the top-down MS technique. The sample can be a biological sample (e.g., a biological fluid). The biological fluid can be blood, serum, plasma, urine, lachrymal fluid, or saliva. The biological fluid can be serum. The top-down MS technique can include a liquid chromatography-mass spectrometry (LC- MS) technique. The top-down MS technique can be electrospray ionization mass spectrometry (ESI-MS). The ESI-MS technique can include a quadrupole time-of-flight (TOF) mass spectrometer.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict between the present specification and a reference mentioned herein, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

Figure 1 is a schematic model of IgA multimers and IgM multimers. The J chain is shown as an oval that completes the coupling of the multimeric forms of IgA and IgM via the blue colored fragment crystallizable region (Fc region) of the heavy chain. The red lines represent disulfide bonds that are also formed in addition to the J chain binding to IgA and IgM.
Figures 2A - 2C contain MS spectra of polyclonal IgA. A) A total ion chromatogram (TIC) of polyclonal IgA in a pooled sample of normal human serum. B) An extracted TIC specific for peaks from a glycosylated J chain (glycoform 2). C) An extracted TIC specific for peaks from a glycosylated J chain (glycoform 1).
Figures 3A - 3B contain MS spectra of different glycoforms of IgA J chains. A) A summed mass spectrum of J chains from the peaks in Figure 2B and 2C. B) A deconvoluted mass spectrum of J chains from the peaks in Figure 2B and 2C.
Figure 4 is a top-down mass spectrum of the +16 charge state from a J chain glycoform fragment (SEQ ID NO:2).
Figure 5 is a top-down mass spectrum of the +16 charge state from a J chain glycoform fragment with labeled glycosylation specific fragments at m/z 168, 204, and 366.
Figures 6A - 6C contain MS spectra of polyclonal IgM. A) A TIC of polyclonal IgM in a pooled sample of normal human serum. B) An extracted TIC specific for peaks from a glycosylated J chain (glycoform 2). C) An extracted TIC specific for peaks from a glycosylated J chain (glycoform 1).
Figures 7A - 7B contain MS spectra of different glycoforms of IgM J chains. A) A summed mass spectrum of J chains from the peaks in Figure 6B and 2C. B) A deconvoluted mass spectrum of J chains from the peaks in Figure 6B and 6C.
Figures 8A - 8C contain MS spectra of monoclonal IgA. A) A TIC of monoclonal IgA in a serum sample from a human multiple myeloma patient. B) An extracted TIC specific for peaks from a glycosylated J chain (glycoform 2). C) An extracted TIC specific for peaks from a glycosylated J chain (glycoform 1).
Figures 9A - 9B contain MS spectra of different glycoforms of IgA J chains. A) A summed mass spectrum of J chains from the peaks in Figure 8B and 8C. B) A deconvoluted mass spectrum of J chains from the peaks in Figure 8B and 8C.
Figures 10A - 10C contain MS spectra of monoclonal IgM. A) A TIC of monoclonal IgM in a serum sample from a human multiple myeloma patient. B) An extracted TIC specific for peaks from a glycosylated J chain (glycoform 2). C) An extracted TIC specific for peaks from a glycosylated J chain (glycoform 1).
Figures 11A - 11B contain MS spectra of different glycoforms of IgM J chains. A) A summed mass spectrum of J chains from the peaks in Figure 8B and 8C. B) A deconvoluted mass spectrum of J chains from the peaks in Figure 8B and 8C.

### DETAILED DESCRIPTION

This document provides methods and materials for identifying and quantifying immunoglobulin J chains and/or identifying the presence or absence, quantity, and/or glycoform(s) of the J chain in a sample using top-down MS. For example, the materials and methods provided herein can be used to identify and quantify the J chain associated with IgA multimers and IgM multimers. In some cases, identification and quantification of the J chain can be used to identify and quantify multimeric IgA immunoglobulins (e.g., IgA dimers) and/or multimeric IgM immunoglobulins (e.g., IgM pentamers).

J chains described herein are detected using top-down MS. The speed, sensitivity, resolution, and robustness of top-down MS make the present methods superior to gel electrophoresis for screening samples for the presence or absence, quantity, and/or glycoform(s) of J chains. A method described herein can include the use of a liquid chromatography MS (LC-MS). In some cases, electrospray ionization MS (ESI-MS) techniques can be used, for example, an electrospray ionization quadrupole time-of-flight MS (ESI-Q-TOF MS) technique. The use of mass over charge (m/z), optionally with additional techniques, such as gel electrophoresis and/or peptide sequencing, provides a more direct assessment of the J chain because it can be used to determine the quantity of the intact J chain and the extent of J chain glycosylation in a single assay.

The methods described herein, also referred to as monoclonal immunoglobulin Rapid Accurate Mass Measurement (miRAMM), can be used to identify and quantify J chains in a sample (e.g., a serum sample) without the need for additional instrumentation or reagents specific to the J chain. The J chain can be identified in the purified sample by its molecular mass. The mean molecular mass for J chains is about 17.5 kDa (17,500 Da). Different J chain glycoforms can have a molecular mass of about 17,400 Da to about 17,900 Da. The presence or absence, quantity, and/or glycoform(s) of J chains can be determined by detecting a J chain amino acid sequence. An exemplary J chain can be a human J chain amino acid sequence as set forth below: 
QEDERIVLVDNKCKCARITSRIIRSSEDPNEDIVERNIRIIVPLNNRENISDPTSPLRT
RFVYHLSDLCKKCDPTEVELDNQIVTATQSNICDEDSATETCYTYDRNKCYTAVV
PLVYGGETKMVETALTPDACYPD (SEQ ID NO:1).
 In some cases, the methods described herein can detect a fragment (e.g., the C-terminus) of a J chain. For example, a J chain can include the amino acid sequence LTPDACYPD (SEQ ID NO:2).

In some cases, a J chain can lack posttranslational modifications (e.g., glycosylation).

In some cases, a J chain can include one or more posttranslational modifications (e.g., glycosylation). A glycosylated J chain can include any appropriate carbohydrate (e.g., deoxyhexose, hexose, N-acetylhexosamine, and sialic acid). A glycosylated J chain can include an N-linked carbohydrate. For example, a glycosylated J chain can include an N-linked carbohydrate at asparagine (N) 49.

The methods described herein can be used to identify and quantify J chains in a sample (e.g., a serum sample) without the need for additional instrumentation or reagents specific to the J chain. The identification and quantification of the J chain can be used to identify and quantify IgA and/or IgM immunoglobulins. For example, an abundance ratio index of intact J chain to intact polyclonal or monoclonal IgA and IgM can be used to determine the amount of multimeric IgA and IgM present in sample (e.g., a serum sample). Thus, the methods described herein provide the ability to identify and quantify the J chain, IgA immunoglobulins, and IgM immunoglobulins, all in the same assay. These methods are useful for screening biological samples for the presence or absence, quantity, and/or glycoform of the J chain, for identifying IgA and/or IgM gammopathies in a patient, for monitoring IgA and/or IgM in a patient, and/or for monitoring treatment of a disorder in a patient.

### Samples and Sample Preparation

The materials and methods for identifying and quantifying J chains as described herein can include any appropriate sample. A sample can be any biological sample, such as a tissue (e.g., adipose, liver, kidney, heart, muscle, bone, or skin tissue) or biological fluid (e.g., blood, serum, plasma, urine, lachrymal fluid, or saliva). The sample can be from a patient that has immunoglobulins, which includes but is not limited to a mammal, e.g. a human, dog, cat, primate, rodent, pig, sheep, cow, horse, bird, reptile, or fish. A sample can also be a man-made reagent, such as a mixture of known composition or a control sample. In some cases, the sample is serum from a human patient.

A sample can be treated to remove components that could interfere with top-down MS. A variety of techniques known to those having skill in the art can be used based on the sample type. Solid and/or tissue samples can be ground and extracted to free the analytes of interest from interfering components. In such cases, a sample can be centrifuged, filtered, and/or subjected to chromatographic techniques to remove interfering components (e.g., cells or tissue fragments). In yet other cases, reagents known to precipitate or bind the interfering components can be added. For example, whole blood samples can be treated using conventional clotting techniques to remove red and white blood cells and platelets. A sample can be deproteinized. For example, a plasma sample can have serum proteins precipitated using conventional reagents such as acetonitrile, KOH, NaOH, or others known to those having ordinary skill in the art, optionally followed by centrifugation of the sample.

Immunoglobulins can be isolated from the samples or enriched (i.e. concentrated) in a sample using standard methods known in the art. Such methods include removing one or more non-immunoglobulin contaminants from a sample. In some cases, the samples can be enriched or purified using immunopurification, centrifugation, filtration, ultrafiltration, dialysis, ion exchange chromatography, size exclusion chromatography, protein A/G or avidin-streptavidin/biotin affinity purification, precipitation, gel electrophoresis, capillary electrophoresis, chemical fractionation (e.g., antibody purification kits, such as Melon Gel Purification), and aptamer techniques. For example, the immunoglobulins can be purified by chemical-based fractionation, e.g.. Melon Gel Chromatography (Thermo Scientific), where Melon Gel resins bind to non-immunoglobulin proteins in a sample and allow immunoglobulins to be collected in the flow-through fraction; or by affinity purification, e.g., by Protein A, Protein G, Protein L, or streptavidin/biotin purification, where immunoglobulins are bound by those proteins at physiologic pH and then released from the proteins by lowering the pH. When serum, plasma, or whole blood samples are used, a sample, such as a 10 - 250 µl sample (e.g., a 50 µl sample), can be directly subjected to Melon Gel, Protein A, Protein G, or Protein L purification. Size exclusion principles such as a TurboFlow column can also be employed to separate the non-immunoglobulin contaminants from a sample. When urine samples are used, a urine sample can be buffered, e.g., a 50 µl urine sample can be diluted first with 50 µl of 50 mM ammonium bicarbonate.

A sample is subject to immunopurification prior to analysis by MS to enrich IgA and/or IgM. For example, immunopurification can separate or enrich immunoglobulin IgA in a sample. For example, immunopurification can separate or enrich IgM in a sample. Immunopurification can involve contacting a sample containing the desired antigen with an affinity matrix including an antibody (e.g. single domain antibody fragments, also referred to as nanobodies) to the antigen covalently attached to a solid phase (e.g., beads such as agarose beads). Antigens in the sample become bound to the affinity matrix through an immunochemical bond. The affinity matrix is then washed to remove any unbound species. The antigen is then removed from the affinity matrix by altering the chemical composition of a solution in contact with the affinity matrix. The immunopurification may be conducted on a column containing the affinity matrix, in which case the solution is an eluent or in a batch process, in which case the affinity matrix is maintained as a suspension in the solution. In some cases, the antibody can be a labelled antibody (e.g. a biotinylated antibody) and a binding partner of the label (e.g., avidin and/or streptavidin) can be attached to the solid phase.

In some embodiments, single domain antibody fragments (SDAFs) with an affinity for immunoglobulins can be used in the immunopurification process. SDAFs can be derived from heavy chain antibodies of non-human sources (e.g., camelids, fish, llama, sheep, goat, rabbit, or mouse), heavy chain antibodies of human sources, and light chain antibodies of humans. SDAFs possess unique characteristics, such as low molecular weight, high physical-chemical stability, good water solubility, and the ability to bind antigens inaccessible to conventional antibodies. For example, IgA immunoglobulins can be immunopurified using anti-IgA camelid nanobodies. For example, IgM immunoglobulins can be immunopurified using anti-IgM camelid nanobodies.

In some embodiments, isolation of immunoglobulins can be performed with an entity other than a traditional antibody-which contains both heavy and light chains (such as those used in IFE and various known clinical immunoassays). Traditional antibodies contain heavy and/or light chains with masses that may overlap with the masses of the immunoglobulins in the sample of interest (e.g., human immunoglobulins). Therefore, these antibodies may interfere in the mass spectra of the patient's immunoglobulins. Single domain antibody fragments (SDAFs) may have masses ranging from 12,500-15,000 Da and, using the methods described herein, may carry a single charge thus generating a signal in the range of 12,500-15,000 m/z, which does not overlap with the signals generated by human heavy chains or light chains. The identification of human light chains and heavy chains by molecular mass can be done as described elsewhere (see, e.g., WO 2015/154052). Thus, in some embodiments, the use of specific isolation of IgA and/or IgM utilizing SDAFs, coupled with mass identification, results in a specific and sensitive method for the detection of J chains.

In some cases, the immunoglobulins (e.g., IgA and/or IgM) are substantially isolated. By "substantially isolated" is meant that the immunoglobulins are at least partially or substantially separated from the sample from which they were provided. Partial separation can include, for example, a sample enriched in the immunoglobulins. Substantial separation can include samples containing at least about 10%, at least about 20%, at least about 30%, at least about 40%, at least about 50%, at least about 60%, at least about 70%, at least about 80%, at least about 90%, at least about 95%, at least about 97%, or at least about 99% by weight of the immunoglobulin.

In some cases, intact immunoglobulins can be further processed to decouple the light chains in a total immunoglobulin sample from the heavy chain immunoglobulins. Decoupling can be achieved by treating the total immunoglobulins with a reducing agent, such as DTT (2,3 dihydroxybutane-1,4-dithiol), DTE (2,3 dihydroxybutane-1,4-dithiol), thioglycolate, cysteine, sulfites, bisulfites, sulfides, bisulfides, TCEP (tris(2-carboxyethyl)phosphine), 2-mercaptoethanol, and salt forms thereof. In some cases, the reducing step is performed at elevated temperature, e.g., in a range from about 30 °C to about 65 °C, such as about 55 °C, in order to denature the proteins. In some cases, the sample is further treated, e.g., by modifying the pH of the sample or buffering the sample. In some cases, the sample can be acidified. In some cases, the sample can be neutralized (e.g., by the addition of a base such as bicarbonate).

Excess reagents and salts can be removed from the samples using methods known to those having ordinary skill in the art.

### Mass Spectrometry Methods

The materials and methods for identifying and quantifying J chains as described herein can include any appropriate top-down MS technique. After sample preparation, a sample is subjected to a top-down MS technique, either directly or after separation on a high performance liquid chromatography column (HPLC). In some cases, LC-MS can be used to analyze the mass spectrum of the ions. For example, the method can be used to identify multiply charged ions (e.g., the +1 ions, +2 ions, +3 ions, +4 ions, +5 ions, +6 ions, +7 ions, +8 ions, +9 ions, +10 ions, +11 ions, +12 ions, +13 ions, +14 ions, +15 ions, +16 ions, +17 ions, +18 ions, +19 ions, +20 ions, +21 ions, and +22 ions), resulting from the J chains in the sample. In some cases, the +16 charged ion is identified and used for further analysis. In some cases, the samples are not fragmented during the top-down MS technique. LC-MS is an analytical technique that combines the physical separation capabilities of liquid chromatography with the mass analysis capabilities of top-down MS, and is suitable for detection and potential identification of chemicals in a complex mixture. Any LC-MS instrument can be used, e.g., the ABSciex 5600 Mass Spectrometer. In some cases, microflowLC-MS can be utilized. Any suitable microflow instrument can be used, e.g., the Eksigent Ekspert 200 microLC. The ion mass spectrum can be analyzed for one or more peaks corresponding to one or more J chains in the sample. For example, one or more ion peaks, e.g., a +16 ion peak, can be examined to identify and monitor the J chains in the sample.

In some cases, ESI-Q-TOF MS can be used to analyze the mass spectrum of a sample, e.g., the mass spectrum of the +16 charge state of the J chain in the sample. ESI MS is a useful technique for producing ions from macromolecules because it overcomes the propensity of these molecules to fragment when ionized. In addition, ESI often produces multiply charged ions, effectively extending the mass range of the analyzer to accommodate the orders of magnitude observed in proteins and other biological molecules. A quadrupole mass analyzer (Q) consists of four cylindrical rods, set parallel to each other. In a quadrupole mass spectrometer, the quadrupole is the component of the instrument responsible for filtering sample ions based on their mass-to-charge ratio (m/z). The time-of-flight (TOF) analyzer uses an electric field to accelerate the ions through the same potential, and then measures the time they take to reach the detector. If the particles all have the same charge, the kinetic energies are identical, and their velocities depend only on their masses. Lighter ions reach the detector first. Any ESI-Q-TOF mass spectrometer can be used, e.g., the ABSciex TripleTOF 5600 quadrupole TOF mass spectrometer. The mass spectrum, e.g., the mass spectrum of multiply charged intact J chain, light chain, and/or heavy chain polypeptide ions, can be analyzed to identify one or more peaks at an appropriate mass/charge expected for the chain. For example, for the J chain, the peaks (e.g., fragment ion peaks) can occur at about 100 - 2500 m/z. In some cases, the peaks can occur at about 150 - 1200 m/z (e.g., about 200 - 1000 m/z for the +16 ion).

The multiply charged ion peaks can be converted to a molecular mass using known techniques. For example, multiply charged ion peak centroids can be used to calculate average molecular mass and the peak area value used for quantification is supplied by a software package. Specifically, multiple ion deconvolution can be performed using the Bayesian Protein Reconstruct software package in the BioAnalyst companion software package in ABSCIEX Analyst TF 1.6. Deconvoluted and multiply charged ions can also be manually integrated using the Manual Integration 33 script in Analyst TF. Providing the molecular mass for the J chain in the sample facilitates sequencing and identification of the J chain in the sample. For example, the methods provided herein can be used to identify and quantify J chains in the sample. In addition, the methods provided herein can be used to compare the relative abundance of the J chains as compared to a control or reference sample. As described herein, the J chain can include the C-terminal amino acid sequence LTPDACYPD (SEQ ID NO:2). The abundance of this J chain sequence can be indicative of the abundance of multimeric IgA and IgM immunoglobulins and therefore is a useful tool for diagnosing and monitoring patients with an IgA gammopathy and/or IgM gammopathy.

In some cases, matrix assisted laser adsorption ionization - time-of-flight (MALDI-TOF) MS can be used to analyze the mass spectrum of a sample. MALDI-TOF MS identifies proteins and peptides as mass charge (m/z) spectral peaks. Further, the inherent resolution of MALDI-TOF MS allows assays to be devised using multiple affinity ligands to selectively purify/concentrate and then analyze multiple proteins in a single assay.

### Methods for Assessing J Chains

The materials and methods provided herein are used for identifying and monitoring J chains. In some cases, the methods provided herein can be used to determine the presence or absence, quantity, and/or glycoform of the J chain. For example, the presence or absence, quantity, and/or glycoform of the J chain can be used for identifying IgA and/or IgM gammopathies in a patient, for monitoring IgA and/or IgM in a patient, and/or for monitoring treatment of a disorder in a patient. For example, an abundance ratio of intact J chain to intact polyclonal or monoclonal IgA or IgM can be used to determine the amount of multimeric IgA or IgM.

The top-down MS based methods disclosed herein can be used to screen a sample (e.g., a biological sample) for the presence, absence, or amount of J chains. In some cases, the top-down MS based methods disclosed herein can be used for detecting J chains in a sample from a patient. In some cases, the top-down MS based methods disclosed herein can be used for detecting IgA immunoglobulins and/or IgM immunoglobulins in a patient. An abundance ratio of intact J chain to intact polyclonal or monoclonal IgA and/or IgM can be used to determine the amount of multimeric IgA immunoglobulins and/or IgM immunoglobulins. For example, the presence of a single J chain can indicate the presence of a single intact IgA dimer (e.g., in an immunopurified IgA sample) or the presence of a single intact IgM pentamer (e.g., in an immunopurified IgM sample). In some cases, the top-down MS based methods disclosed herein can be used for diagnosing a patient having an IgA gammopathy and/or IgM gammopathy.

The top-down MS based methods disclosed herein can include subjecting a sample having one or more immunoglobulins to a top-down MS assay. The sample can be pretreated to isolate or enrich immunoglobulins present in the sample. The immunoglobulin light chains can be decoupled from the immunoglobulin heavy chains prior to the top-down MS analysis. The spectrum obtained from the assay can then be used to identify J chains in the sample. In some cases, the abundance (e.g., quantity) of J chains can be determined by converting the peak areas of one or more of the identified peaks into a molecular mass.

The ratios, relative abundance, and/or glycoform of the J chains can be used to diagnose various disorders associated with abnormal production of IgA and/or IgM including, but not limited to, polyclonal gammopathies (e.g., IgA gammopathies and/or IgM gammopathies), autoimmune disorders, infectious disorders, and/or inflammatory disorders. A disorder can be chronic or acute (e.g., due to infection). When the disorder is a gammopathy, the gammopathy can include an overproduction of immunoglobulins or a deficiency of immunoglobulins. When the disorder is a gammopathy, the gammopathy can be a monoclonal gammopathy or a polyclonal gammopathy. Examples of disorders associated with abnormal production of IgA and/or IgM include, without limitation, multiple myeloma, asymptomatic or smoldering multiple myeloma, solitary plasmacytoma, monoclonal gammopathy of undetermined significance, B-cell leukemia (e.g., B-cell chronic lymphocytic leukemia), Waldenstrom macroglobulinemia, non-secretory myeloma, selective IgA deficiency, selective IgM deficiency, celiac disease, IgA nephropathy, autoimmune disorders, and neurologic diseases.

In some cases, the ratios and relative abundance of the J chains can be compared to a reference value or a control sample. For example, a reference value can be a relative abundance of J chains, IgA, and/or IgM in a healthy patient (e.g., a healthy human). For example, a control sample can be a sample (e.g., serum) obtained from one or more healthy patients (e.g., healthy humans).

In some cases, the methods provided herein can be used to confirm a diagnosis made by current methods such as gel electrophoresis. For example, if a negative result is obtained from gel electrophoresis, the present methods can be used as a secondary test to confirm or counter such results. In some cases, the diagnosis provided herein can be confirmed using such standard methods.

In some cases that are not claimed as such, the methods provided herein can be used for treating a patient having a gammopathy. For example, after diagnosing the subject as having a gammopathy as described herein (e.g., based on the presence or absence, quantity, and/or glycoform of the J chain), the methods can include administering to the patient a therapeutic agent to treat the gammopathy (e.g., a therapeutically effective amount) and/or performing a treatment (e.g., a plasma exchange or a Stem cell transplant). The therapeutic agent can be any appropriate therapeutic agent. For example, when the gammopathy is a monoclonal gammopathy of undetermined significance, the therapeutic agent can be a bisphosphonate. Non-limiting examples of bisphosphonates include alendronate (e.g., BINOSTO^{®}, FOSAMAX^{®}, risedronate (e.g., ACTONEL^{®}, ATELVIA^{®}), ibandronate (e.g., BONIVA^{®}) and zoledronic acid (e.g., RECLAST^{®}, ZOMETA^{®}). For example, when the gammopathy is a B-cell chronic lymphocytic leukemia, the therapeutic agent can be chlorambucil, fludarabine (e.g., FLUDARA^{®}), cyclophosphamide (e.g., CYTOXAN^{®}), bendamustine, cyclophosphamide, doxorubicin, vincristine (e.g., ONCOVIN^{®}), prednisonea, chlorambucil, obinutuzumab, ofatumumab, pentostatin (e.g., NIPENT^{®}), alemtuzumab (e.g., CAMPATH^{®}), fludarabine, a monoclonal antibody (e.g., rituximab), or any combinations thereof. For example, when the gammopathy is Waldenstroms macroglobulinemia, the therapeutic agent can be bortezomib, thalidomide, fludarabine, dexamethasone, chlorambucil, cyclophosphamide, doxorubicin, vincristine, prednisone, a monoclonal antibody (e.g., rituximab), a purine nucleoside analog (e.g., cladribine), ibrutinib, or any combinations thereof. For example, when the gammopathy is a non- secretory myeloma, the therapeutic agent can be thalidomide, bortezomib, lenalidomide, carfilzomib, pomalidomide, or any combinations thereof. In some cases, after diagnosing the subject as having a gammopathy, the method can include administering to the subject a therapeutically effective amount of a therapeutic agent to treat the gammopathy and one or more of a plasma exchange and a stem cell transplant (e.g., an autologous peripheral blood Stem cell transplantation).

In some cases, the methods provided herein can also be used for monitoring a patient. For example, the top-down MS based methods disclosed herein can be used for monitoring a IgA gammopathy and/or a IgM gammopathy in a patient. The top-down MS based methods disclosed herein can include providing a first sample and a second sample of the subject. For example, the top-down MS based methods disclosed herein can include providing a first sample of the subject before the treatment and a second sample of the subject during or after the treatment. The first and second samples can be pretreated to isolate or enrich immunoglobulins present in the first and second samples. The immunoglobulin light chains in the first and second samples can be decoupled from the immunoglobulin heavy chains prior to the top-down MS analysis. The spectrum obtained from the assay can then be used to identify J chains in the first and second samples. In some cases, the relative abundance of J chains in the first and second samples can be determined by converting the peak areas of one or more of the identified peaks into a molecular mass. The presence or absence of J chains can be determined in the first and second samples. A decrease (or loss) of the amount of J chains indicates that the IgA gammopathy and/or IgM gammopathy in the patient has been reduced (or eliminated); while an increase in the amount of J chains indicates that the IgA gammopathy and/or IgM gammopathy in the patient has increased. In cases where a first sample of the subject is before the treatment and a second sample of the subject is during or after the treatment, the presence or absence of J chains is determined before and after the treatment and compared. A decrease (or loss) of the amount of J chains indicates that the treatment may be effective for the subject; while an increase or no change in the amount of J chains indicates that the treatment may be ineffective for the subject. For example, the amount of J chains in a first sample and in a second sample can be determined, and the amount of J chains in the first sample can be compared to the amount of J chains and the second sample. For example, the concentration of J chains in a first sample and in a second sample can be determined, and the concentration of J chains in the first sample can be compared to the amount of J chains and the second sample.

The invention will be further described in the following examples, which do not limit the scope of the invention described in the claims.

### EXAMPLES

### Example 1: Mass Spectrometry to Identify J Chain Fragments in Patient Serum

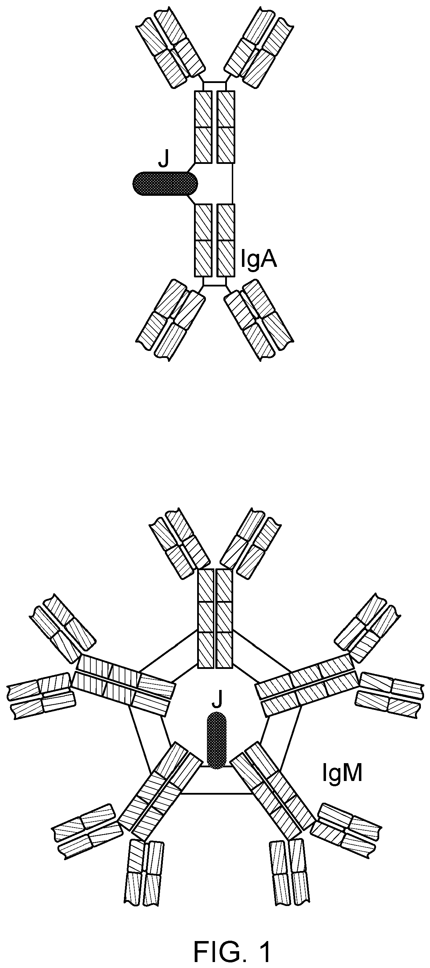

IgA and IgM immunoglobulins to form multimers as shown in Figure 1. In circulation IgA typically exists as a combination of monomers and dimers while IgM exists as a pentamer. In order for multimers of both IgA and IgM to form they must be connected by a 17.5 kDa protein called the J chain (see, e.g.. Figure 1).

### Methods

A volume of 50 µL of normal control pooled serum was added to 20 µL of anti-IgA or anti-IgM nanobody beads. The serum was allowed to incubate with the nanobody beads for 45 minutes. The beads were washed with 1 mL of PBS 3 times each time removing and discarding the supernatant. The beads were then washed with water 1 time. The water was removed and 50 µL of 5% acetic acid containing 50 mM TCEP was added to the beads to elute the purified immunoglobulins. This elute was then analyzed by microflow LC-ESI-Q-TOF MS using a SCIEX 5600 mass spectrometer.

### Results

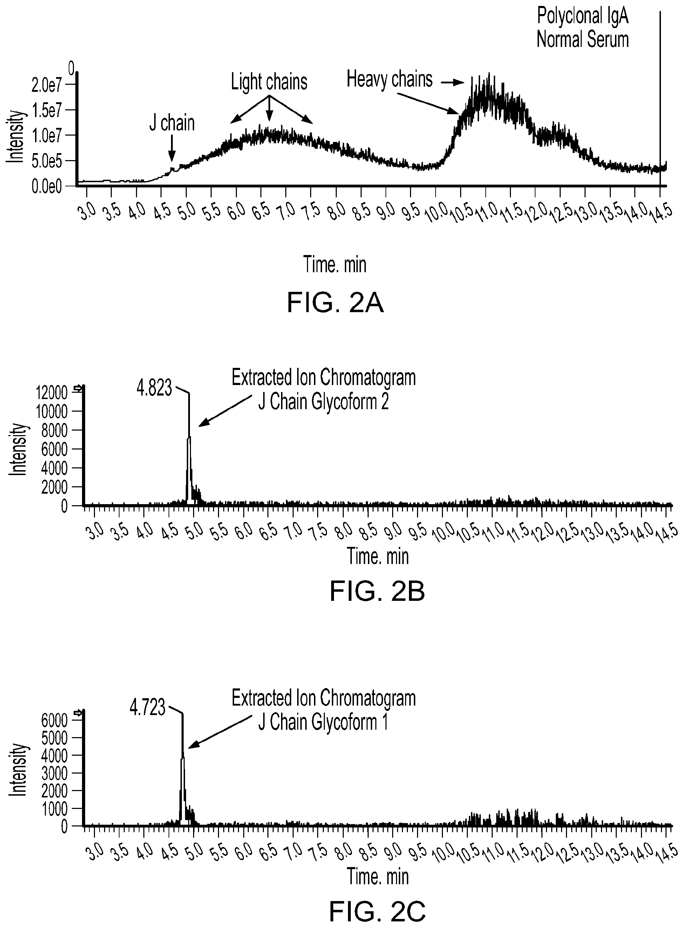

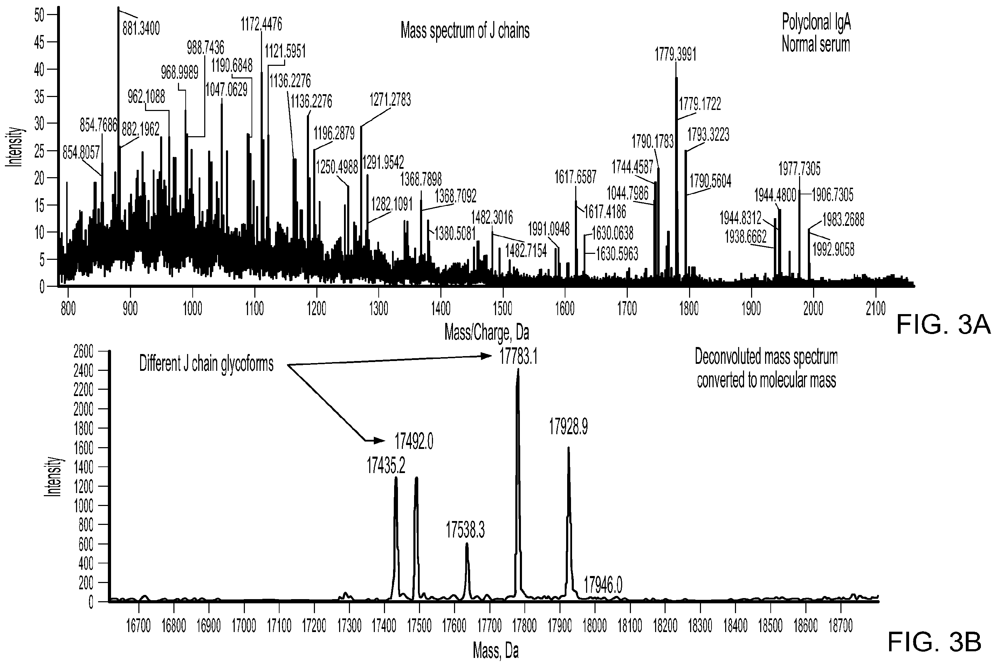

IgA was purified using camelid nanobody beads from normal pooled serum and subjected to MS (Figure 2A). Extracted ion chromatograms specific for peaks from the J chain (Figure 2B and 2C) show the relative abundance of the J chain compared to all the IgA molecules purified from the serum. Not all serum IgA is multimeric (i.e., contains J chains), thus the relative abundance of the J chain is low compared to the abundance of the IgA light chains and heavy chains. Mass spectra were obtained for the different glycoforms of the J chains shown in Figures 2B and 2C (Figure 3A). The different glycoforms of the J chain and relative abundance of each are clearly visible in the deconvoluted mass spectrum (Figure 3B).

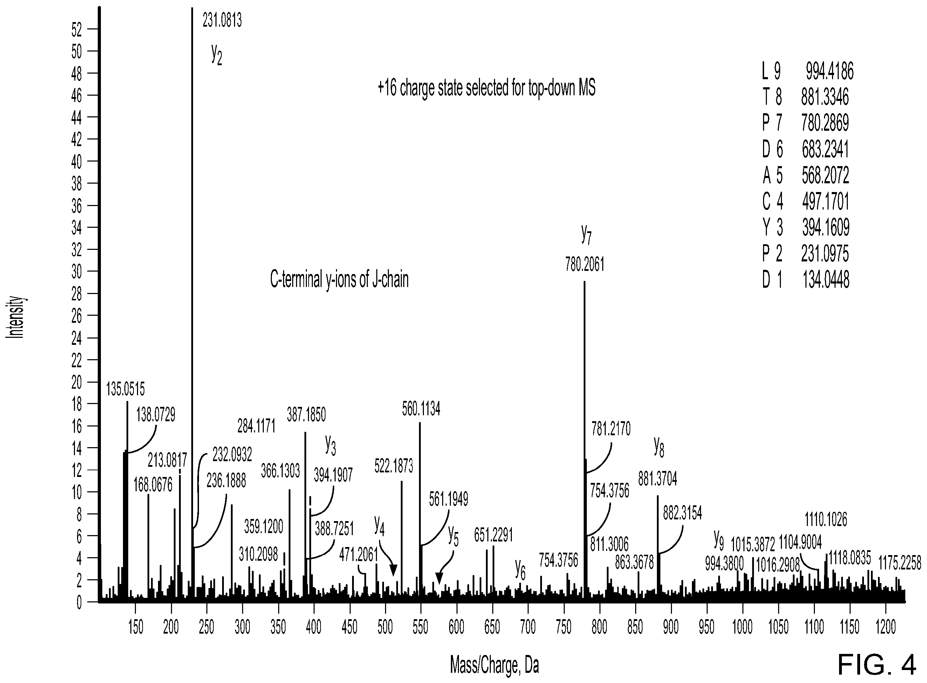

To confirm the identity of the J chain in the sample, a top-down fragment ion mass spectrum was derived from the +16 charge state for a specific J chain glycoform. The spectrum shows the y-ion series from the known amino acids from the C-terminus of the J chain (Figure 4).

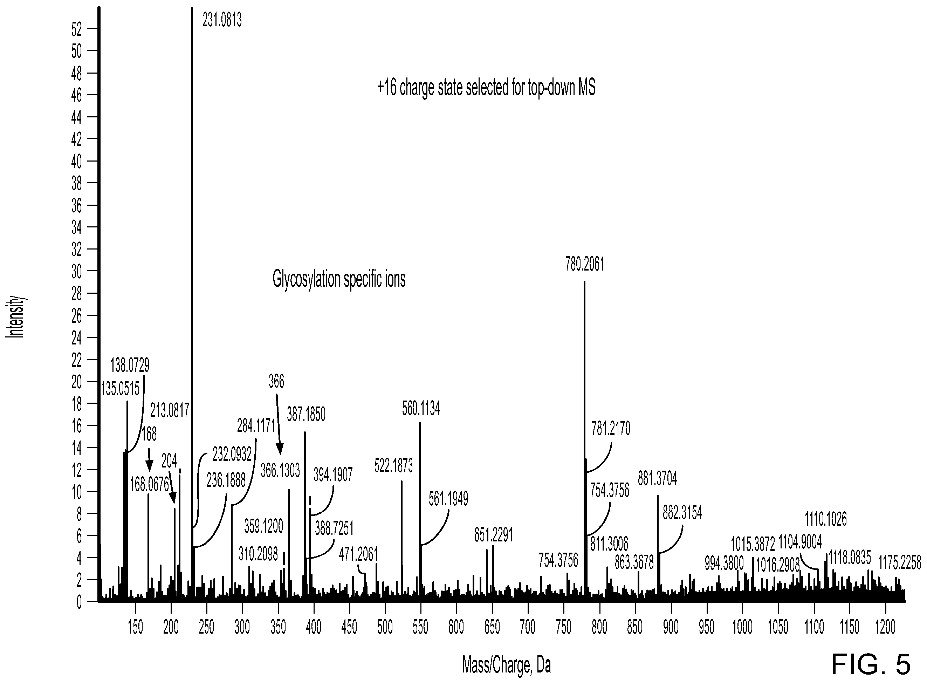

To examine whether the J chains observed are glycosylated, a top-down fragment ion mass spectrum was derived from the +16 charge state for a specific J chain glycoform. The spectrum shows the glycosylation specific fragment ions that can only come from a glycosylated protein (Figure 5).

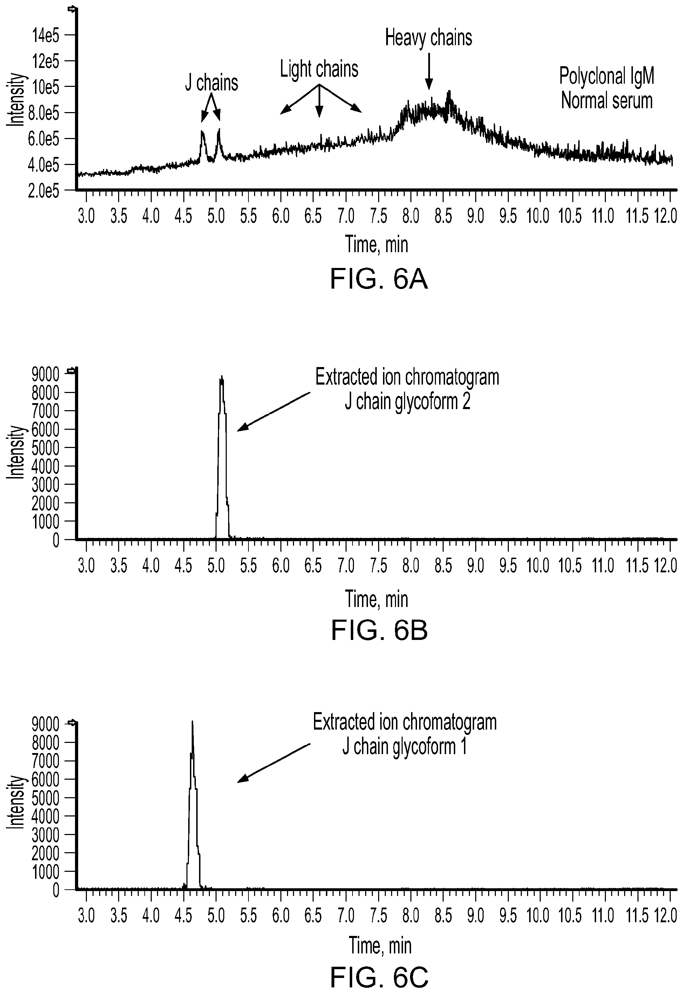

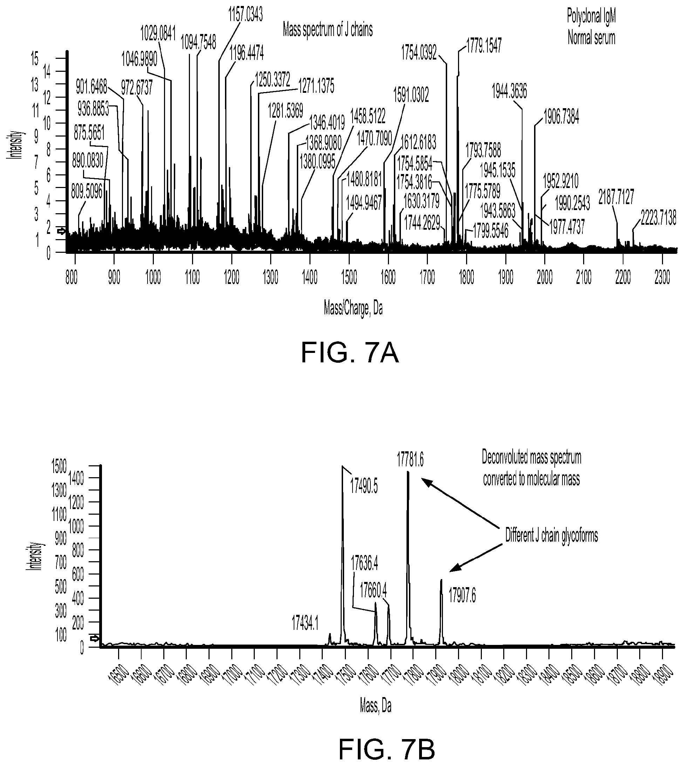

IgM was purified using camelid nanobody beads from normal pooled serum and subjected to top-down MS (Figure 6A). Extracted ion chromatograms specific for peaks from the J chain (Figure 6B and 6C) show the relative abundance of the J chain compared to all the IgM molecules purified from the serum. All serum IgM is multimeric (i.e., contains J chains), thus the relative abundance of the J chain is larger compared to IgA. This also confirmed that the relative abundance of the J chain to IgM is greater as compared to the relative abundance of the J chain to IgA since the same serum was used for each experiment. Mass spectra were obtained for the different glycoforms of the J chains shown in Figures 6B and 6C (Figure 7A). The different glycoforms of the J chain and relative abundance of each are clearly visible in the deconvoluted mass spectrum (Figure 7B).

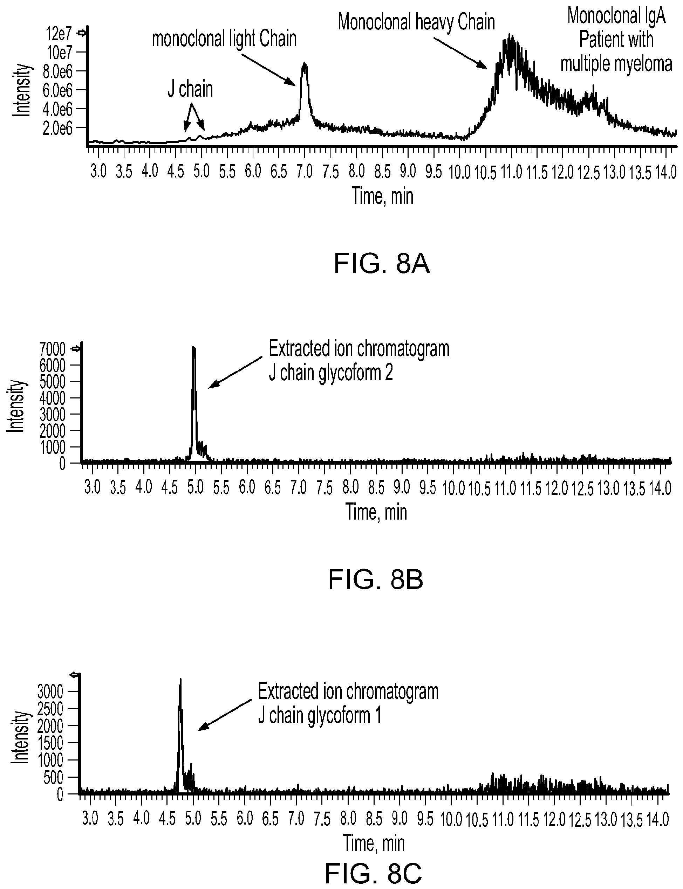

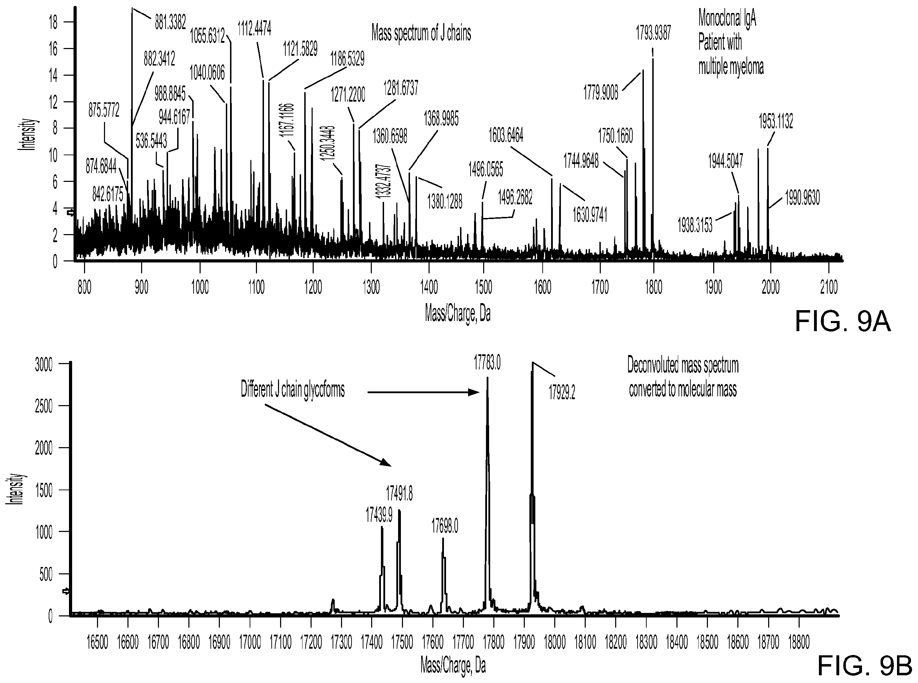

IgA was purified using camelid nanobody beads from serum from a patient with multiple myeloma and subjected to top-down MS (Figure 8A). Extracted ion chromatograms specific for peaks from the J chain (Figure 8B and 8C) show the relative abundance of the J chain compared to the monoclonal IgA light chain and heavy chain purified from serum. Mass spectra were obtained for the different glycoforms of the J chains shown in Figures 8B and 8C (Figure 9A). The different glycoforms of the J chain and relative abundance of each are clearly visible in the deconvoluted mass spectrum (Figure 9B). The relative abundance of each IgA J chain glycoform in MM was nearly identical to the abundances observed in normal serum (compare Figure 3B and Figure 9B).

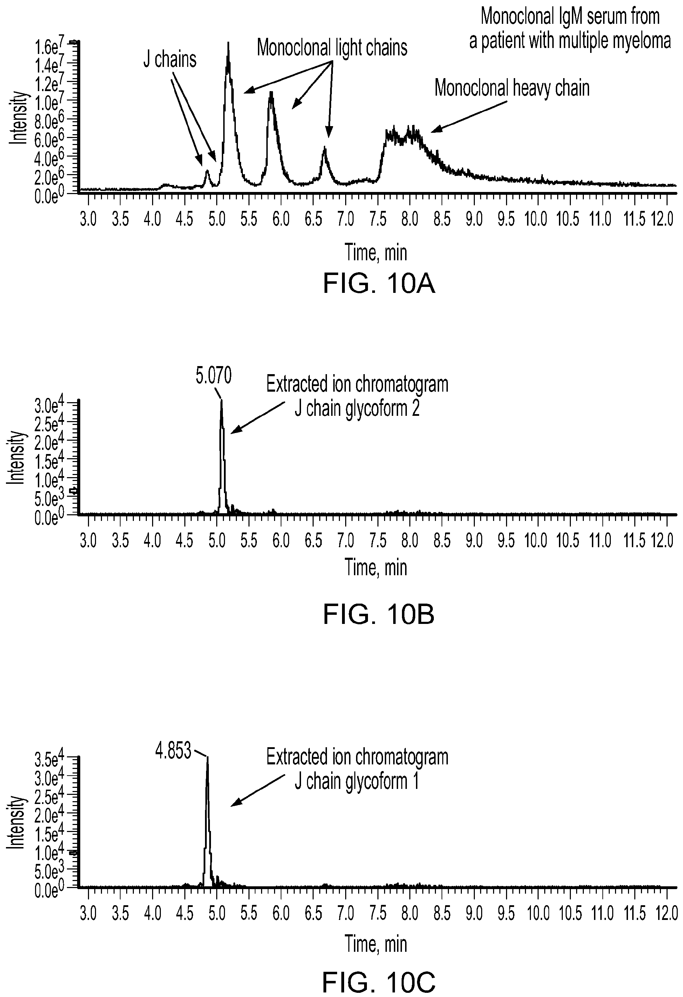

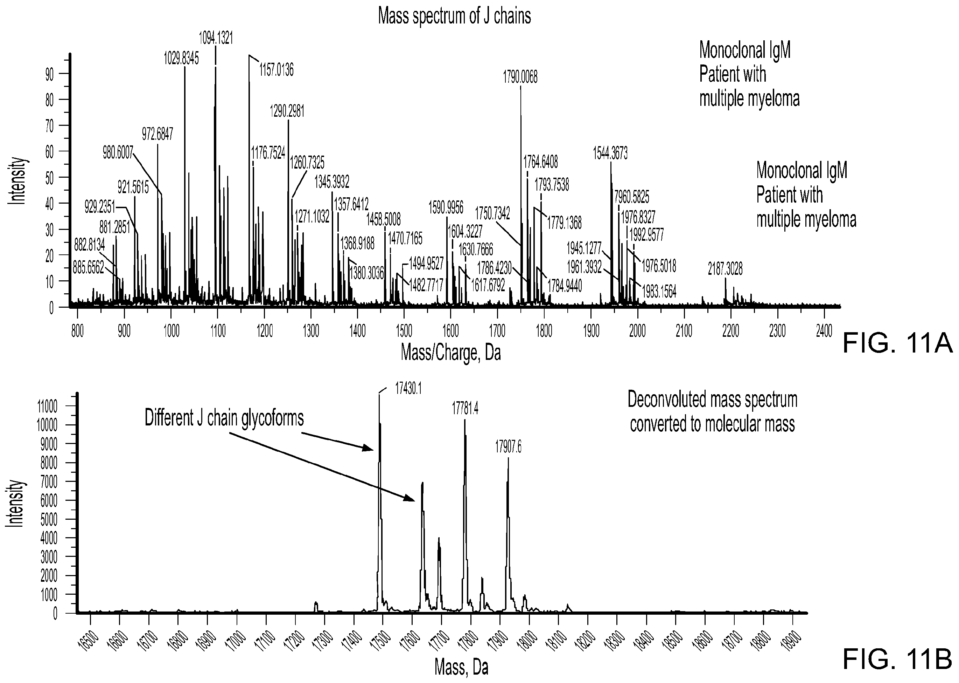

IgM was purified using camelid nanobody beads from serum from a patient with multiple myeloma and subjected to top-down MS (Figure 10A). Extracted ion chromatograms specific for peaks from the J chain (Figure 10B and 10C) show the relative abundance of the J chain compared to the monoclonal IgM light chain and heavy chain purified from serum. Mass spectra were obtained for the different glycoforms of the J chains shown in Figures 10B and 10C (Figure 11A). The different glycoforms of the J chain and relative abundance of each are clearly visible in the deconvoluted mass spectrum (Figure 11B). The relative abundance of each IgM J chain glycoform in MM was nearly identical to the abundances observed in normal serum (compare Figure 7B and Figure 11B).

## Claims

1. A method for identifying J chains in a sample, the method comprising:
providing a sample comprising immunoglobulins;
immunopurifying IgA immunoglobulins and/or IgM immunoglobulins from the sample;
subjecting the immunopurified immunoglobulins to a top-down mass spectrometry technique to obtain a mass spectrum of the sample; and
identifying the presence of J chains based on the multiply charged ion peaks in the spectrum corresponding to the J chains.

2. A method for quantifying the J chains in a sample, the method comprising:
providing a sample comprising immunoglobulins;
immunopurifying IgA immunoglobulins comprising using an anti-human IgA antibody and/or IgM immunoglobulins comprising using an anti-human IgM antibody from the sample;
subjecting the immunopurified immunoglobulins to a top-down mass spectrometry technique to obtain a mass spectrum of the sample;
identifying the presence of J chains based on the multiply charged ion peaks in the spectrum corresponding to the J chains; and
converting the peak area of the identified peaks to a molecular mass to quantify the J chains in the sample.

3. The method of claim 1 or claim 2, comprising immunopurifying IgA from the sample; or immunopurifying IgM from the sample.

4. The method of any one of claims 1 to 3 wherein said immunopurifying comprises using a non-human antibody,
preferably wherein said non-human antibody is a camelid antibody, a cartilaginous fish antibody, llama, sheep, goat, rabbit, or a mouse antibody,
more preferably wherein said antibody is a single domain antibody fragment.

5. The method of claim 2, comprising immunopurifying IgA immunoglobulins from the sample, and further comprising quantifying the IgA immunoglobulins,
preferably wherein said IgA immunoglobulins are multimeric IgA immunoglobulins.

6. The method of claim 5, wherein said quantifying comprises determining the ratio of J chains to IgA immunoglobulins.

7. The method of claim 2, comprising immunopurifying IgM immunoglobulins from the sample, and further comprising quantifying the IgM immunoglobulins,
preferably, wherein said IgM immunoglobulins are multimeric IgM immunoglobulins.

8. The method of claim 7, wherein said quantifying comprises determining the ratio of J chains to IgM immunoglobulins.

9. The method of any one of claims 1 to 8, wherein said immunoglobulins are not fragmented during the mass spectrometry technique.

10. The method of any one of claims 1 to 9, wherein said sample is a biological sample,
preferably wherein said biological sample is a biological fluid,
more preferably wherein said biological fluid is selected from the group consisting of blood, serum, plasma, urine, lachrymal fluid, and saliva.

11. The method of any one of claims 1 to 10, wherein said mass spectrometry technique comprises a liquid chromatography-mass spectrometry (LC-MS) technique,
Preferably wherein the mass spectrometry technique is electrospray ionization mass spectrometry (ESI-MS),
more preferably wherein the ESI-MS technique comprises a quadrupole time-of-flight (TOF) mass spectrometer.

12. The method of any one of claims 1 to 11, wherein the J chains lack a posttranslational modification or
comprise a post-translational modification,
preferably wherein said post-translational modification is glycosylation.

13. A method for diagnosing a disorder in a patient, wherein said disorder is associated with abnormal production of IgA immunoglobulins and/or IgM immunoglobulins, the method comprising the use of the method of claims 1-12;
determining the ratio of J chains to IgA immunoglobulins and/or the ratio of J chains to IgM immunoglobulins in the sample; and
comparing the ratio to a reference value.

14. A method of monitoring a treatment of a disorder in a patient, wherein said disorder is associated with abnormal production of IgA immunoglobulins and/or IgM immunoglobulins, the method comprising:
providing an initial sample comprising immunoglobulins from the patient, wherein said initial sample has been obtained from the patient prior to the treatment;
providing one or more secondary samples comprising immunoglobulins, wherein said one or more secondary samples have been obtained from the patient during the treatment, after the treatment, or both;
immunopurifying IgA immunoglobulins comprising using an anti-human IgA antibody and/or IgM immunoglobulins from the samples using an anti-human IgM antibody;
subjecting the immunopurified immunoglobulins to a top-down mass spectrometry technique to obtain a mass spectrum of the samples;
identifying the presence of J chains in said samples based on the multiply charged ion peaks in the spectrum corresponding to the J chains;
determining the ratio of J chains to IgA immunoglobulins and/or the ratio of J chains to IgM immunoglobulins in the samples; and
comparing the ratios from the initial sample and the one or more secondary samples,
preferably wherein said disorder is selected from the group consisting of multiple myeloma, asymptomatic or smoldering multiple myeloma, solitary plasmacytoma, monoclonal gammopathy of undetermined significance, B-cell chronic lymphocytic leukemia, Waldenstrom macrogloblinemia, non-secretory myeloma, selective IgA deficiency, selective IgM deficiency, celiac disease, IgA nephropathy, autoimmune disorders, and neurologic diseases.

15. The method of any one of claims 13 or 14, wherein said patient is a mammal, preferably wherein said mammal is a human.

## Patentansprüche

1. Verfahren zum Identifizieren von J-Ketten in einer Probe, wobei das Verfahren Folgendes umfasst:
Bereitstellen einer Probe, die Immunglobuline umfasst;
Immunaufreinigen von IgA-Immunglobulinen und/oder IgM-Immunglobulinen aus der Probe;
Anwenden einer Top-Down-Massenspektrometrietechnik auf die immungereinigten Immunglobuline, so dass ein Massenspektrum der Probe erhalten wird; und
Identifizieren des Vorliegens von J-Ketten anhand der Peaks mehrfach geladener Ionen im Spektrum, die den J-Ketten entsprechen.

2. Verfahren zum Quantifizieren der J-Ketten in einer Probe, wobei das Verfahren Folgendes umfasst:
Bereitstellen einer Probe, die Immunglobuline umfasst;
Immunaufreinigen von IgA-Immunglobulinen, was Verwenden eines Antikörpers gegen menschliches IgA umfasst, und/oder von IgM-Immunglobulinen, was Verwenden eines Antikörpers gegen menschliches IgM umfasst, aus der Probe;
Anwenden einer Top-Down-Massenspektrometrietechnik auf die immungereinigten Immunglobuline, so dass ein Massenspektrum der Probe erhalten wird; und
Identifizieren des Vorliegens von J-Ketten anhand der Peaks mehrfach geladener Ionen im Spektrum, die den J-Ketten entsprechen; und
Umrechnen der Peak-Fläche der identifizierten Peaks in eine Molekülmasse zur Quantifizierung der J-Ketten in der Probe.

3. Verfahren nach Anspruch 1 oder Anspruch 2, umfassend Immunaufreinigen von IgA aus der Probe oder Immunaufreinigen von IgM aus der Probe.

4. Verfahren nach einem der Ansprüche 1 bis 3, wobei das Immunaufreinigen Verwenden eines nichtmenschlichen Antikörpers umfasst,
bevorzugt wobei es sich bei dem nichtmenschlichen Antikörper um einen Camelidae-Antikörper, ein Knorpelfisch-Antikörper, einen Lama-, Schaf-, Ziege-, Kaninchen- oder einen Maus-Antikörper handelt,
bevorzugter wobei es sich bei dem Antikörper um ein Einzeldomäne-Antikörperfragment handelt.

5. Verfahren nach Anspruch 2, umfassend Immunaufreinigen von IgA-Immunglobulinen aus der Probe und ferner umfassend Quantifizieren der IgA-Immunglobuline,
bevorzugt wobei es sich bei den IgA-Immunglobulinen um multimere IgA-Immunglobuline handelt.

6. Verfahren nach Anspruch 5, wobei das Quantifizieren Bestimmen des Verhältnisses von J-Ketten zu IgA-Immunglobulinen umfasst.

7. Verfahren nach Anspruch 2, umfassend Immunaufreinigen von IgM-Immunglobulinen aus der Probe und ferner umfassend Quantifizieren der IgM-Immunglobuline,
bevorzugt wobei es sich bei den IgM-Immunglobulinen um multimere IgM-Immunglobuline handelt.

8. Verfahren nach Anspruch 7, wobei das Quantifizieren Bestimmen des Verhältnisses von J-Ketten zu IgM-Immunglobulinen umfasst.

9. Verfahren nach einem der Ansprüche 1 bis 8, wobei die Immunglobuline während der Massenspektrometrietechnik nicht fragmentiert werden.

10. Verfahren nach einem der Ansprüche 1 bis 9, wobei es sich bei der Probe um eine biologische Probe handelt, bevorzugt wobei es sich bei der biologischen Probe um eine biologische Flüssigkeit handelt,
bevorzugter wobei die biologische Flüssigkeit aus der Gruppe bestehend aus Blut, Serum, Plasma, Urin, Tränenflüssigkeit und Speichel ausgewählt ist.

11. Verfahren nach einem der Ansprüche 1 bis 10, wobei die Massenspektrometrietechnik eine LC-MS(Liquid Chromatography-Mass Spectrometry)-Technik umfasst,
bevorzugt wobei es sich bei der Massenspektrometrietechnik um ESI-MS (Electrospray Ionization Mass Spectrometry) handelt,
bevorzugter wobei die ESI-MS-Technik ein Quadrupol-Flugzeit(TOF)-Massenspektrometer umfasst.

12. Verfahren nach einem der Ansprüche 1 bis 11, wobei den J-Ketten eine posttranslationale Modifikation fehlt oder die J-Ketten
eine posttranslationale Modifikation umfassen,
bevorzugt wobei es sich bei der posttranslationalen Modifikation um Glykosylierung handelt.

13. Verfahren zur Diagnose einer Störung bei einem Patienten, wobei die Störung mit einer anomalen Produktion von IgA-Immunglobulinen und/oder IgM-Immunglobulinen assoziiert ist, wobei das Verfahren die Verwendung des Verfahrens nach Anspruch 1-12,
Bestimmen des Verhältnisses von J-Ketten zu IgA-Immunglobulinen und/oder des Verhältnisses von J-Ketten zu IgM-Immunglobulinen in der Probe und
Vergleichen des Verhältnisses mit einem Referenzwert umfasst.

14. Verfahren zum Überwachen einer Behandlung einer Störung bei einem Patienten, wobei die Störung mit einer anomalen Produktion von IgA-Immunglobulinen und/oder IgM-Immunglobulinen assoziiert ist, wobei das Verfahren Folgendes umfasst:
Bereitstellen einer Immunglobuline umfassenden ersten Probe von dem Patienten, wobei die erste Probe vor der Behandlung vom Patienten erhalten wurde;
Bereitstellen einer oder mehrerer Immunglobuline umfassender sekundärer Proben, wobei die eine oder mehreren sekundären Proben während der Behandlung oder/und nach der Behandlung erhalten wurden;
Immunaufreinigen von IgA-Immunglobulinen, was Verwenden eines Antikörpers gegen menschliches IgA umfasst, und/oder von IgM-Immunglobulinen aus den Proben, wobei ein Antikörper gegen menschliches IgM verwendet wird;
Anwenden einer Top-Down-Massenspektrometrietechnik auf die immungereinigten Immunglobuline, so dass ein Massenspektrum der Proben erhalten wird;
Identifizieren des Vorliegens von J-Ketten in den Proben anhand der Peaks mehrfach geladener Ionen im Spektrum, die den J-Ketten entsprechen.
Bestimmen des Verhältnisses von J-Ketten zu IgA-Immunglobulinen und/oder des Verhältnisses von J-Ketten zu IgM-Immunglobulinen in den Proben; und
Vergleichen der Verhältnisse aus der ersten Probe und den eine oder mehreren sekundären Proben,
bevorzugt wobei die Störung aus der Gruppe bestehend aus multiplem Myelom, asymptomatischem oder schwelendem multiplem Myelom, solitärem Plasmazytom, monoklonaler Gammopathie unbestimmter Signifikanz, chronischer lymphatischer B-Zell-Leukämie, Waldenström-Makrogloblinämie, nicht sekretorischem Myelom, selektivem IgA-Mangel, selektivem IgM-Mangel, Zöliakie, IgA-Nephropathie, Autoimmunerkrankungen und neurologischen Erkrankungen ausgewählt ist.

15. Verfahren nach Anspruch 13 oder Anspruch 14, wobei es sich bei dem Patienten um einen Säuger handelt, bevorzugt wobei es sich bei dem Säuger um einen Menschen handelt.

## Revendications

1. Procédé pour identifier des chaînes J dans un échantillon, le procédé comprenant :
la fourniture d'un échantillon comprenant des immunoglobulines ;
l'immunopurification d'immunoglobulines IgA et/ou d'immunoglobulines IgM de l'échantillon ;
la soumission des immunoglobulines immunopurifiées à une technique de spectrométrie de masse descendante pour obtenir un spectre de masse de l'échantillon ; et
l'identification de la présence de chaînes J sur la base des pics ioniques à charges multiples dans le spectre correspondant aux chaînes J.

2. Procédé pour quantifier les chaînes J dans un échantillon, le procédé comprenant :
la fourniture d'un échantillon comprenant des immunoglobulines ;
l'immunopurification d'immunoglobulines IgA comprenant l'utilisation d'un anticorps anti-IgA humaine et/ou d'immunoglobulines IgM comprenant l'utilisation d'un anticorps anti-IgM humaine provenant de l'échantillon ;
la soumission des immunoglobulines immunopurifiées à une technique de spectrométrie de masse descendante pour obtenir un spectre de masse de l'échantillon ;
l'identification de la présence de chaînes J sur la base des pics ioniques à charges multiples dans le spectre correspondant aux chaînes J ; et
la conversion de l'aire de pic des pics identifiés en une masse moléculaire pour quantifier les chaînes J dans l'échantillon.

3. Procédé selon la revendication 1 ou la revendication 2, comprenant l'immunopurification d'IgA à partir de l'échantillon ; ou l'immunopurification d'IgM à partir de l'échantillon.

4. Procédé selon l'une quelconque des revendications 1 à 3, dans lequel ladite immunopurification comprend l'utilisation d'un anticorps non humain,
de préférence, dans lequel ledit anticorps non humain est un anticorps de camélidé, un anticorps de poisson cartilagineux, un anticorps de lama, de mouton, de chèvre, de lapin ou de souris,
plus préférablement dans lequel ledit anticorps est un fragment d'anticorps à domaine unique.

5. Procédé selon la revendication 2, comprenant l'immunopurification d'immunoglobulines IgA de l'échantillon, et comprenant en outre la quantification des immunoglobulines IgA,
de préférence, dans lequel lesdites immunoglobulines IgA sont des immunoglobulines IgA multimériques.

6. Procédé selon la revendication 5, dans lequel ladite quantification comprend la détermination du rapport des chaînes J sur les immunoglobulines IgA.

7. Procédé selon la revendication 2, comprenant l'immunopurification d'immunoglobulines IgM de l'échantillon, et comprenant en outre la quantification des immunoglobulines IgM,
de préférence, dans lequel lesdites immunoglobulines IgM sont des immunoglobulines IgM multimériques.

8. Procédé selon la revendication 7, dans lequel ladite quantification comprend la détermination du rapport des chaînes J sur les immunoglobulines IgM.

9. Procédé selon l'une quelconque des revendications 1 à 8, dans lequel lesdites immunoglobulines ne sont pas fragmentées pendant la technique de spectrométrie de masse.

10. Procédé selon l'une quelconque des revendications 1 à 9, dans lequel ledit échantillon est un échantillon biologique,
de préférence dans lequel ledit échantillon biologique est un fluide biologique,
plus préférablement, dans lequel ledit fluide biologique est choisi dans le groupe constitué par le sang, le sérum, le plasma, l'urine, le fluide lacrymal, et la salive.

11. Procédé selon l'une quelconque des revendications 1 à 10, dans lequel ladite technique de spectrométrie de masse comprend une technique de chromatographie liquide-spectrométrie de masse (LC-MS),
de préférence, dans lequel la technique de spectrométrie de masse est la spectrométrie de masse à ionisation par électrospray (ESI-MS),
plus préférablement, la technique ESI-MS comprend un spectromètre de masse à temps de vol (TOF) quadripolaire.

12. Procédé selon l'une quelconque des revendications 1 à 11, dans lequel les chaînes J ne présentent pas une modification post-traductionnelle ou
comprend une modification post-traductionnelle,
préférablement où ladite modification post-traductionnelle est une glycosylation.

13. Procédé pour diagnostiquer un trouble chez un patient, dans lequel ledit trouble est associé à une production anormale d'immunoglobulines IgA et/ou d'immunoglobulines IgM, le procédé comprenant l'utilisation du procédé selon les revendications 1-12 ; la détermination du rapport des chaînes J sur les immunoglobulines IgA et/ou du rapport des chaînes J sur les immunoglobulines IgM dans l'échantillon ; et
la comparaison du rapport à une valeur de référence.

14. Procédé de suivi d'un traitement d'un trouble chez un patient, dans lequel ledit trouble est associé à une production anormale d'immunoglobulines IgA et/ou d'immunoglobulines IgM, le procédé comprenant :
la fourniture d'un échantillon initial comprenant des immunoglobulines du patient, dans lequel ledit échantillon initial a été obtenu du patient avant le traitement ;
la fourniture d'un ou plusieurs échantillons secondaires comprenant des immunoglobulines, dans lequel ledit ou lesdits échantillons secondaires ont été obtenus du patient pendant le traitement, après le traitement, ou les deux ;
l'immunopurification des immunoglobulines IgA comprenant l'utilisation d'un anticorps anti-IgA humaine et/ou d'immunoglobulines IgM provenant des échantillons comprenant l'utilisation d'un anticorps anti-IgM humaine ;
la soumission des immunoglobulines immunopurifiées à une technique de spectrométrie de masse descendante pour obtenir un spectre de masse des échantillons ;
l'identification de la présence de chaînes J dans lesdits échantillons sur la base des pics ioniques à charges multiples dans le spectre correspondant aux chaînes J ;
la détermination du rapport de chaînes J sur les immunoglobulines IgA et/ou le rapport de chaînes J sur les immunoglobulines IgM dans les échantillons ; et
la comparaison des rapports de l'échantillon initial et du ou des échantillons secondaires,
préférablement, ledit trouble étant choisi dans le groupe constitué un myélome multiple, un myélome multiple asymptomatique ou latent, un plasmocytome solitaire, une gammapathie monoclonale de signification indéterminée, une leucémie lymphoïde chronique à cellules B, une macrogloblinémie de Waldenström, un myélome non sécrétant, un déficit sélectif en IgA, un déficit sélectif en IgM, une maladie cœliaque, une néphropathie à IgA, des maladies auto-immunes et des maladies neurologiques.

15. Procédé selon l'une quelconque des revendications 13 ou 14, dans lequel ledit patient est un mammifère, de préférence dans lequel ledit mammifère est un humain.
